Europäisches Patentamt

European Patent Office

Office européen des brevets

(11) Veröffentlichungsnummer : **0 342 404 B1**

(12) **EUROPÄISCHE PATENTSCHRIFT**

(45) Veröffentlichungstag der Patentschrift :
07.10.92 Patentblatt 92/41

(21) Anmeldenummer : **89107605.1**

(22) Anmeldetag : **27.04.89**

(51) Int. Cl.⁵ : **A61M 1/02**

(54) **Vorrichtung zur Trennung von Komponenten einer Flüssigkeit, insbesondere von Gesamtblut.**

(30) Priorität : **07.05.88 DE 3815643**

(43) Veröffentlichungstag der Anmeldung :
**23.11.89 Patentblatt 89/47**

(45) Bekanntmachung des Hinweises auf die
Patenterteilung :
**07.10.92 Patentblatt 92/41**

(84) Benannte Vertragsstaaten :
**DE FR GB IT NL**

(56) Entgegenhaltungen :
**EP-A- 0 175 618**
**EP-A- 0 213 469**
**DE-A- 3 012 228**
**DE-A- 3 417 892**
**US-A- 4 354 116**

(73) Patentinhaber : **NPBI Nederlands**
**Produktielaboratorium voor**
**Bloedtransfusieapparatuur en**
**Infusievloeistoffen B.V.**
**Runde ZZ41**
**NL-7881 HM Emmer Compascuum (NL)**

(72) Erfinder : **Gänshirt, Karlheinz, Dr. Dipl.-Chem.**
**August-Bebel-Strasse 34**
**W-6072 Dreieich (DE)**
Erfinder : **Handel, Klaus Dieter, Dr. Dipl.-Ing.**
**Pallaswiesenstrasse 14**
**W-6100 Darmstadt (DE)**
Erfinder : **Walker, Wolfram, Dr. Dipl.-Chem.**
**Thomastrasse 8**
**W-6074 Rödermark (DE)**

(74) Vertreter : **Masch, Karl Gerhard, Dr. Dipl.-Phys.**
**et al**
**Patentanwälte Andrejewski, Honke & Partner**
**Theaterplatz 3 Postfach 10 02 54**
**W-4300 Essen 1 (DE)**

**Beschreibung**

Die Erfindung betrifft eine Vorrichtung zur aufeinanderfolgenden Gewinnung von in mindestens zwei räumlich getrennten Bereichen vorliegenden Komponenten einer Flüssigkeit, insbesondere stabilisiertes Gesamtblut, welche in einem flexiblen Beutel mit mindestens einer Auslassleitung enthalten ist.

Bisher standen zur getrennten Gewinnung von Komponenten einer Flüssigkeit, insbesondere Gesamtblut in zentrifugierten Beuteln, Vorrichtungen zur Verfügung, bei welchen die die Flüssigkeit bzw. das Gesamtblut enthaltenden Beutel zwischen einer senkrecht angeordneten stationären Platte und einer dazu beweglichen, an der Unterkante drehbaren Platte zusammengedrückt werden.

Die hierzu benötigte Kraft wird mittels Federn erzeugt. Solche als normale Plasmaquetschen bekannte Vorrichtungen weisen jedoch erhebliche Nachteile auf. Zum einen muss der Andruckvorgang kontinuierlich überwacht werden, um bei Erreichen einer bestimmten Komponentenschicht am oberen Rand den Druckvorgang zu reduzieren bzw. zu beenden. Zum anderen nimmt die Federkraft während des Zusammendrückens des Beutels ab. Folglich ist, um eine stärker viskose Schicht, wie z.B. die Buffy-coat-Schicht im Blut auszupressen, ein höherer Druck als der mittels Federkraft erzeugbare notwendig. Daher müssen solche Schichten meist mit manueller Unterstützung entfernt werden. Zusätzlich sind nur Beutel mit einer oberen Auslassleitung hier verwendbar.

Eine Verbesserung dieser normalen Plasmaquetsche wurde durch Einsatz eines Zellsensors erzielt. Der Abquetschvorgang braucht dadurch nicht mehr überwacht zu werden. (Siehe beispieleweise US-A-4.354.116 oder EP-A-0175168.) Die durch die Federkraft auftretenden Druckinhomogenitäten und damit verbundene Nachteile bleiben jedoch weiterhin vorhanden.

Eine wesentliche Verbesserung der obengenannten Vorrichtungen wurde durch Geräte mit automatisch arbeitendem Antriebselement erzielt.

In der DE-A-30 12 228 wird eine Vorrichtung beschrieben, bei der der Andrückvorgang hydraulisch, pneumatisch oder hydropneumatisch erfolgt. Sensoren zur Ermittlung der verschiedenen Komponentenschichten erlauben einen automatischen Abquetschvorgang.

In der DE-A-34 17 892 wird eine ebenfalls automatisch angetriebene Vorrichtung beschrieben, bei welcher das Abquetschelement zwischen einer oberen und einer unteren Andruckplatte angeordnet ist. Es müssen hier also drei Andruckelemente separat angesteuert werden.

Bei beiden Vorrichtungen sind stationäre Platte und Andruckplatte parallel angeordnet. Daher muss der Beutel solange zwischen den Platten manuell festgehalten werden, bis er nach Anlegen des Druckes durch den beginnenden Pressvorgang fixiert ist. Aufgrund der tropfenartigen Form erfolgt hierbei schnell eine Verformung des Beutels und dadurch eine Verwirbelung der Grenzschicht des Beutelinhalts, so dass das Auspressen einer reinen Komponentenschicht nicht ohne weiteres gewährleistet ist. Da die auf die stationäre Platte ausgeübte Kraft relativ hoch ist (ca. 50 kp oder ca. 500N), sind relativ stabile Plattenkonstruktionen erforderlich.

Der Erfindung liegt daher die Aufgabe zugrunde, eine Vorrichtung zur getrennten Gewinnung von Komponenten einer Flüssigkeit bereitzustellen, wobei der Beutel nicht manuell fixiert werden muss und keine schnelle anfängliche Beuteldeformierung und somit eine geringere Durchmischung der zentrifugierten Schichten erfolgt. Zusätzlich soll der Pressvorgang automatisch unter einem konstanten Auspressdruck erfolgen. Durch Einsatz geeigneter Abtastvorrichtungen und Beutel mit mindestens einer, vorzugsweise zwei Auslassleitungen soll insgesamt eine schnell arbeitende rationelle und apparativ einfache Vorrichtung insbesondere zur Herstellung von reinen Blutpräparaten ermöglicht werden.

Diese Aufgabe wird erfindungsgemäss durch die im Hauptanspruch gekennzeichnete Vorrichtung gelöst.

Das wesentliche Merkmal besteht darin, dass die stationäre Platte und die Andruckplatte nicht parallel, sondern im Ausgangszustand im Winkel zueinander angeordnet sind und der Drehpunkt der Andruckplatte in der Schnittlinie beider Plattenflächen unterhalb des Beutels liegt, und zwar in einem Abstand von etwa 30% der Länge der Andruckplatte. Dadurch ist der Beutel vor Anlegen des Auspressdrucks durch einfaches Einlegen so fixierbar, dass keine schnelle Verformung bei Beginn des Abquetschvorgangs erfolgt. Aufgrund des grösseren Querschnitts im oberen Beutelteil wird die Gefahr der Grenzschichtverwirbelung auch gegen Ende der Präparation im Vergleich zu parallel angeordneten Platten erheblich reduziert. Ferner ist das Mehrfachbeutelverbindungssystem, z.B. das Abbrechteil, am Beutelkopf aufgrund der grösseren Öffnungsweite gegenüber paralleler Plattenanordnung sehr leicht entfernbar. Die um etwa 30 % gegenüber der stationären Platte verkürzte Andruckplatte ermöglicht die Verwendung von Beuteln mit einer zusätzlichen unteren Auslassleitung. Automatischer Antrieb mittels eines über einen Hebelarm mit der Andruckplatte starr verbundenen Antriebselements sowie entsprechende Abtasteinrichtungen der erfindungsgemässen Vorrichtung erlauben insgesamt eine rationelle, einfache und schnelle Bereitstellung z.B. reiner Blutpräparate.

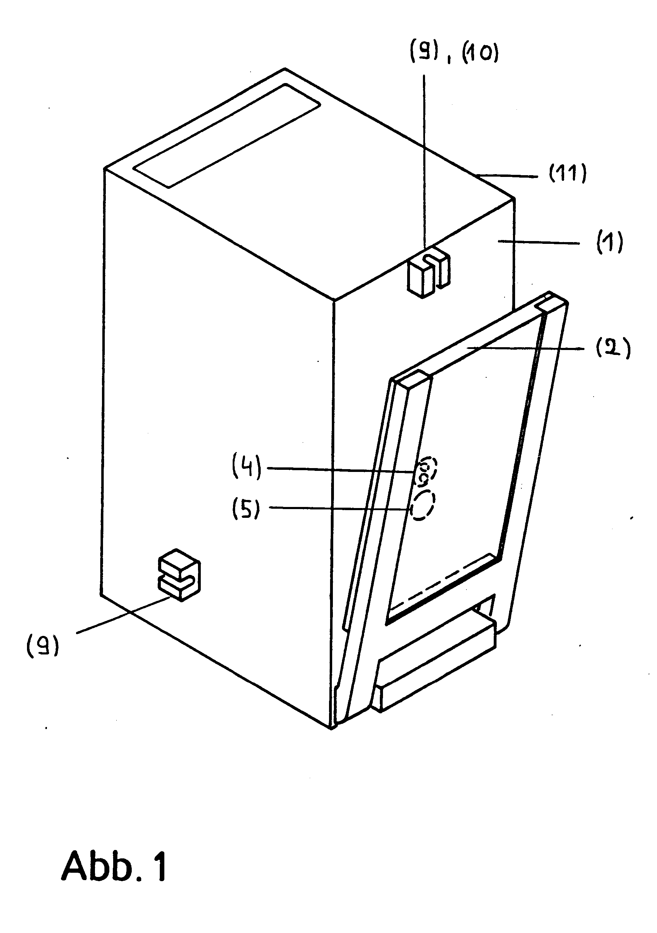

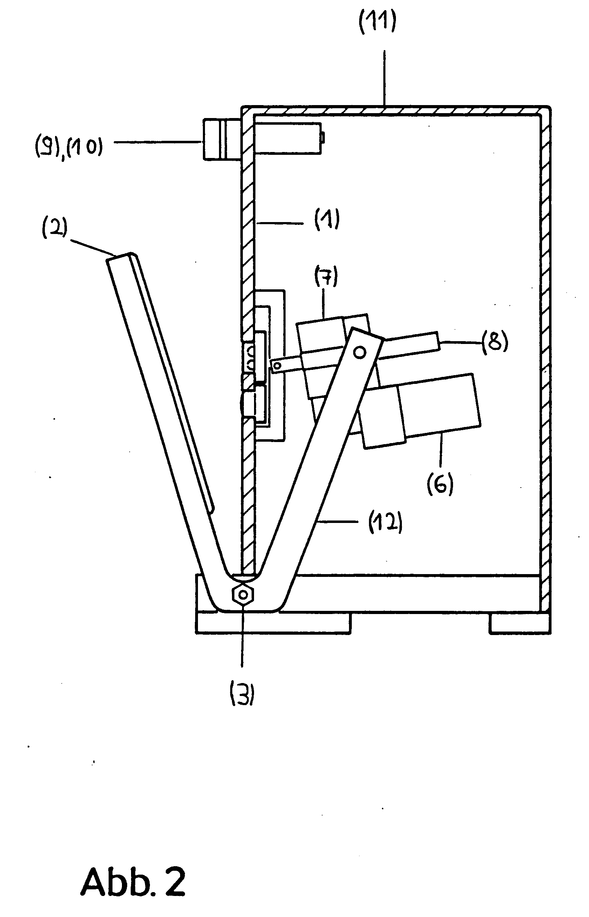

Abbildungen 1 und 2 zeigen eine bevorzugte Ausführungsform der erfindungsgemässen Vorrichtung.

EP 0 342 404 B1

Abb. 1 (perspektivische Vorderansicht)

Die erfindungsgemässe Vorrichtung besteht aus einem die Antriebsvorrichtung enthaltenden Gehäuse (11) mit (stationärer) Frontplatte (1) und einer dazu im Ausgangszustand im Winkel angeordneten Andruckplatte (2). Diese Andruckplatte (2) kann aus Kunststoff bestehen und ist in einem Metallrahmen verankert. Die Schnittlinie beider Plattenflächen liegt unterhalb des Beutels in einem Abstand von etwa 30% der Länge der Andruckplatte (2). In dieser Schnittlinie ist der Drehpunkt (3) der Andruckplatte (2) um mehr als 30% unterhalb der Länge der Andruckplatte bzw. des Beutels angeordnet. Dadurch können zusätzliche Schlauchanschlüsse für Beutel mit mehr als einer Auslasseinrichtung, z.B. solche wie in der EP-A-0 213 469 beschriebene Beutel-Konstruktionen mit Oben- und Untenauslass, in dem Zwischenraum zwischen Drehpunkt (3) und Beutel fixiert werden, so dass ein komplettes Zusammendrücken des Beutels durch das Schlauchsystem nicht behindert werden kann.

Durch die erfindungsgemässe Anordnung des Drehpunktes (3) der Andruckplatte (2) weiter unterhalb des Beutels als bei den bekannten Vorrichtungen ist die starke Beutelformveränderung beim Einlegen zu Beginn des Abquetschvorgangs deutlich reduziert. Die Gefahr der Verwirbelung der Grenzschicht ist erheblich vermindert.

Aufgrund der Winkelstellung beider Platten kann der Beutel immer gleichartig positioniert werden. Dabei ist die nach oben gerichtete Komponente der Strömung der leichteren Komponente geringer aufgrund des grösseren Querschnitts dieses Beutelbereichs. Die Gefahr des Mitreissens der schweren Komponente wird folglich verringert.

Die Antriebsvorrichtung besteht aus einem über einen Hebelarm (12) mit der Andruckplatte (2) starr verbundenen Antriebselement. Durch beispielsweise elektrisch oder hydraulisch erzeugte Kraft wird die Andruckplatte (2) bewegt. Vorzugsweise kann das Antiebselement, bestehend aus dem Motor (6), dem Getriebe (7) und der Antriebsspindel (8) an der Frontplatte (1) angeordnet sein. Dadurch wirkt auf die Frontplatte (1) von aussen und innen nahezu die gleiche Kraft. Die Frontplatte (1) ist somit nur mehr geringen Drehmomenten im Verhältnis zum Gerät selbst ausgesetzt. Aufgrund dieser geringen Druckbelastung kann die Frontplatte (1) aus einem Material geringer Dicke beschaffen sein, da keine hohe Kraft aufgefangen werden muss.

Als Abtastvorrichtung können Schlauchdetektoren (10), wie sie in der GB-A-1 537 096 beschrieben sind, oder auch ein in der Mitte der Frontplatte angordneter Beuteldetektor (4) verwendet werden. Dadurch wird die während des Abquetschvorgangs ansteigende Grenzschicht erkannt und entsprechende damit gekoppelte Schlauchklemmen (9) geschlossen, sobald das Auspressen einer Komponente beendet ist.

Die erfindungsgemässe Vorrichtung kann zusätzlich mit einem Drucksensor (5) ausgestattet sein, welcher unterhalb des Beuteldetektors (4) an der Frontplatte (1) angeordnet ist und das Antriebselement steuert. Dadurch wird eine konstante Andruckkraft gewährleistet. Bei eventuellem Blockieren einer Auslassleitung wird dann die Andruckskraft automatisch reduziert, so dass kein unerwünschter Überdruck auftreten kann.

Weiterhin kann die beanspruchte Vorrichtung mit einem Endschalter ausgestattet sein, der bei einem vorgewählten Plattenabstand bzw. restlichem Beutelvolumen die Klemme(n) des Geräts schliesst.

Diese Ausführung ist vor allem für Beutel mit einem oberen und einem unteren Abgang geeignet. Dabei steuert ein Grenzschichtdetektor die obere Klemme. Er kann im Beutelbereich an der Frontplatte (1) oder im Schlauchbereich (10) angeordnet sein. Wird ein Beuteldetektor (4) gewählt, so wird die obere Klemme geschlossen, sobald im Detektor das Ansteigen der Grenzschicht durch das schnellere Abfliessen der leichteren Komponente festgestellt wird. Die schwerere Komponente fliesst dann nach unten, so dass die Grenzschicht wieder absinkt. Sobald im Detektor eine bestimmte Höhe dieser Grenzschicht ermittelt wird, wird die obere Klemme wieder geöffnet. Daduch wird während des gesamten Abquetschvorganges die Grenzschicht auf einer durch den Detektor (4) definierten Höhe gehalten. Bei Erreichen eines vorgewählten Plattenabstandes bzw. Beutelvolumens wird die Klemme bzw. werden beide Klemmen geschlossen und der Vorgang ist beendet.

Wird ein Schlauchdetektor (10) gewählt, der im oberen Schlauchbereich angeordnet ist, so wird die obere Klemme bei komplettem Abpressen der leichteren Komponente geschlossen. Die untere Komponente wird dann bis zu einem vorgegebenen Plattenabstand bzw. Beutelvolumen abgepresst.

Abb.2

Abbildung 2 zeigt eine Seitenansicht der erfindungsgemässen Vorrichtung.

Im Gehäuse (11) mit Frontplatte (1) ist die Antriebsvorrichtung angeordnet. Der Motor (6) und das Getriebe (7) sind über die Antriebsspindel (8) an der Frontplatte (1) befestigt und mit der Andruckplatte (2) über den Hebelarm (12) starr verbunden. In der Mitte der Frontplatte (1) ist ein Beuteldetektor (4) und unterhalb diesem ein Drucksensor (5) angeordnet. Am oberen Ende der Frontplatte (1) sind Schlauchklemmen (9) bzw. ein Schlauchdetektor (10) angeordnet.

3

Die Andruckplatte (2) ist mehr als 30% kürzer als die Frontplatte (1). Die Schnittlinie beider Plattenflächen und der Drehpunkt (3) der Andruckplatte (2) liegen also unterhalb des Beutels.

Erfindungsgemäss wird der das Fluid enthaltende Beutelvorzugsweise mit 2 Auslassleitungen - zwischen die Platten (1) und (2) so eingesetzt, dass der Raum unterhalb seines Endpunktes frei ist und z.B. für die zweite Auslassleitung genutzt wird, ohne dass das Auspressen des Beutels behindert wird.

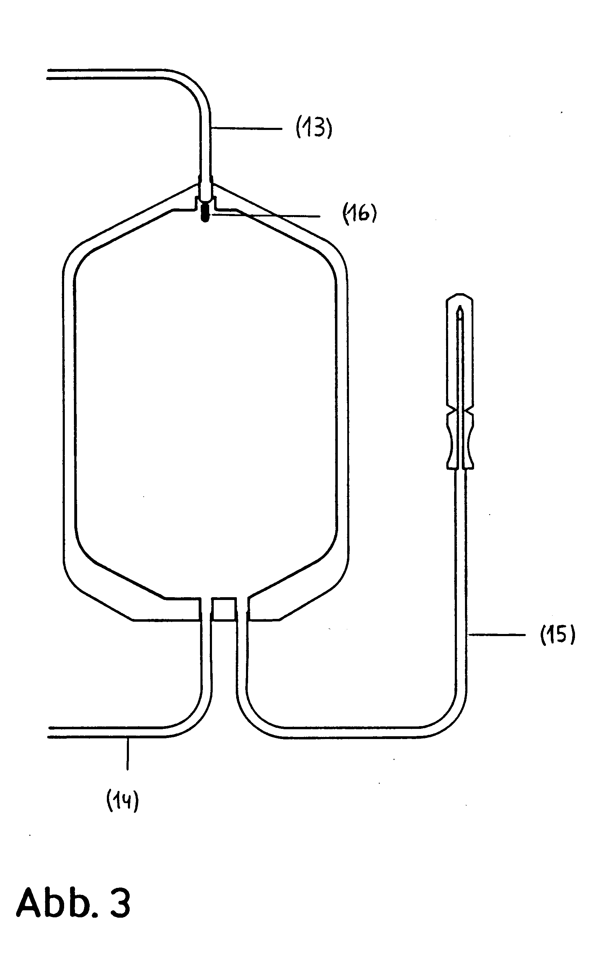

Ein für die erfindungsgemässe Vorrichtung besonders bevorzugter Beutel ist in Abb. 3 dargestellt.

Dieser Beutel weist eine obere Auslassleitung (13) sowie ihr gegenüber eine untere Auslassleitung (14) auf. Er enthält keine Anschlussstutzen und weist nur ein Mehrfachbeutelverbindungssystem, z.B. Abbrechteil (16) auf, welches am oberen Beutelteil angeordnet ist.

Die Auslassleitung (14) ist auf derselben Seite wie der Füll- bzw. Blutentnahmeschlauch (15) des Beutels angeordnet.

Durch diese Beutelkonstruktion wird gewährleistet, dass während des Zentrifugierens das Abbrechelement (16) immer oben liegt und somit nicht vorzeitig abbrechen kann. Beim Öffnen dieses obenliegenden Abbrechteils (16) werden die nach der Zentrifugation getrennten Schichten nicht aufgewirbelt, wie dies bei einem Abbrechteil in der unteren Auslassleitung leicht möglich ist. Zusätzlich werden hierbei keine unerwünschten Blutzellen im Beutelkopf zurückgehalten, was im Falle eines vorhandenen Anschlussstutzens leicht erfolgen kann.

Darüberhinaus wird aus gleichem Grund eine Kontamination im Beutelkopf vermieden, da die Zuleitung (15) ihm gegenüber auf derselben Seite wie die Auslassleitung (14) angeordnet ist.

Der Beutel kann auf die geschilderte Weise in der erfindungsgemässen Vorrichtung durch einfaches Einlegen positioniert und vollständig ausgepresst werden.

Ein wesentlicher Vorteil der beanspruchten Vorrichtung besteht darin, dass durch die erfindungsgemässe Anordnung der verkürzten Andruckplatte (2) und der Frontplatte (1) die nach DIN-Vorschriften standardisierten Beutel mit einem definierten Füllvolumen immer in die gleiche Position eingelegt werden können. Eine zusätzliche manuelle Fixierung bis zu Beginn des Auspressvorgangs, wie es bei der parallelen Anordnung notwendig ist, entfällt. Zusätzlich ist die Gefahr der anfänglich schnellen Beuteldeformation und der damit verbundenen unerwünschten Grenzschichtverwirbelung erheblich vermindert. Durch die erfindungsgemässe Verkürzung der Andruckplatte (2) gegenüber der Frontplatte (1) wird ein vollständiges Auspressen des Beutels gewährleistet, das nicht durch zusätzlich angebrachte Auslassleitungen wie beispielsweise bei Beuteln mit Oben- und Untenauslass behindert werden kann.

Darüber hinaus ist erfindungsgemäss die Gefahr der Grenzschichtverwirbelung auch gegen Ende der Präparation erheblich reduziert aufgrund des grösseren Querschnitts des oberen Beutelteils.

Die durch das Auspressen des Beutelinhalts auf die Frontplatte (1) wirkende Kraft wird gegenüber Vorrichtungen mit parallel angeordneten Platten kompensiert, da durch die Anordnung des Antriebselements nahezu die gleiche Kraft von aussen wie von innen auf die Frontplatte (1) wirkt. Insgesamt können somit dünnere Platten als für die bisher bekannten Vorrichtungen verwendet werden.

Die erfindungsgemässe Vorrichtung kann beispielsweise für die getrennte Gewinnung von Gesamtblutkomponenten, wie z.B. die Leukopherese, Plasmapherese, Thrombopherese u.ä. verwendet werden, wobei vorzugsweise Beutel mit mehr als einer Auslassleitung eingesetzt werden können.

## Patentansprüche

1. Vorrichtung zur Gewinnung von in mindestens zwei räumlich getrennten Bereichen vorliegenden heterogenen Komponenten einer Flüssigkeit, insbesondere stabilisiertes Gesamtblut, in einem flexiblen durchsichtigen Beutel mit mindestens einer Auslassleitung (14), bestehend aus einer Frontplatte (1), die Teil des Gehäuses (11) ist, einer relativ zur Frontplatte (1) schwenkbaren Andruckplatte (2), einer Antriebsvorrichtung und einer Abtasteinrichtung, dadurch gekennzeichnet, dass der Drehpunkt (3) der Andruckplatte (2) unterhalb des Beutels im Schnittpunkt beider Plattenoberflächen liegt, und zwar in einem Abstand von über 30% der Länge der Andruckplatte (2), und die Antriebsvorrichtung an einen starr mit der Andruckplatte (2) verbundenen Hebelarm (12) angreift.

2. Vorrichtung gemäss Anspruch 1, dadurch gekennzeichnet, dass die Abtasteinrichtung ein optischer Detektor (4,10) ist, der eine Einrichtung (9) zum Absperren von mindestens einer der Auslasseinrichtungen betätigt, wenn eine bestimmte Komponentenschicht ein vorgegebenes Niveau im Beutel erreicht.

3. Vorrichtung gemäss Anspruch 2, dadurch gekennzeichnet, dass der optische Detektor (4) im Beutelbereich an der Frontplatte (1) angeordnet ist.

4. Vorrichtung gemäss Anspruch 2, dadurch gekennzeichnet, dass der Detektor (10) im Schlauchbereich angeordnet ist.

5. Vorrichtung gemäss Anspruch 1, dadurch gekennzeichnet, dass zusätzlich ein Endschalter vorgesehen ist.

6. Vorrichtung gemäss Anspruch 1, dadurch gekennzeichnet, dass die Antriebsvorrichtung aus einem hydraulisch oder elektrisch betriebenen Antriebselement besteht.

7. Vorrichtung gemäss Anspruch 6, dadurch gekennzeichnet, dass das Antriebselement an der Frontplatte (1) angeordnet ist.

8. Vorrichtung gemäss einem der Ansprüche 1-7, dadurch gekennzeichnet, dass in der Mitte der Frontplatte (1) ein Drucksensor (5) angeordnet ist.

9. Beutel mit Zuleitung - bzw Ableitungsschläuchen am Kopf und Boden, zur Verwendung in einer Vorrichtung gemäss den Ansprüchen 1-8, dadurch gekennzeichnet, dass eine Einlassleitung (13) am oberen Ende - also dem Drehpunkt (3) der Vorrichtung abgewandt - und eine Auslassleitung (14) sowie der Füll- bzw. Blutentnahmeschlauch (15) am unteren Beutelteil - also dem Drehpunkt (3) der Vorrichtung zugewandt - angeordnet sind und nur ein Mehrfachbeutelverbindungssystem (16) am oberen Ende des Beutels angebracht ist und kein Auslassstutzen vorhanden ist.

## Claims

1. A device for obtaining heterogeneous components, present in at least two spatially separated regions, of a liquid, particularly stabilised whole blood, in a flexible transparent bag with at least one outlet line (14), the device comprising a front plate (1) which is part of the housing (11), a pressure plate (2) which can pivot relative to the front plate (1), a drive device and a scanning device, characterised in that the pivot (3) of the pressure plate (2) is located at a distance of more than 30% of the length of the contact plate (2) below the bag at the point of intersection of the surfaces of the two plates, and the drive device acts upon a lever arm (12) which is rigidly attached to the pressure plate (2).

2. A device according to Claim 1, characterised in that the scanning device is an optical detector (4, 10) which actuates a device (9) for closing at least one of the outlet devices if a certain component layer reaches a predetermined level in the bag.

3. A device according to Claim 2, characterised in that the optical detector (4) is disposed on the front plate (1) in the region of the bag.

4. A device according to Claim 2, characterised in that the detector (10) is disposed in the region of the flexible tubing.

5. A device according to Claim 1, characterised in that a limit switch is provided in addition.

6. A device according to Claim 1, characterised in that the drive device comprises a hydraulically or electrically operated drive element.

7. A device according to Claim 6, characterised in that the drive element is disposed on the front plate (1).

8. A device according to any one of Claims 1 to 7, characterised in that a pressure sensor (5) is disposed in the centre of the front plate (1).

9. A bag with flexible inlet or outlet tubes at the top and the bottom, for use in a device according to Claims 1 to 8, characterised in that an inlet line (13) is disposed at the upper end, i.e. remote from the pivot (3) of the device, and an outlet line (14) and also the flexible tubing for filling or for withdrawing blood (15) are disposed on the lower part of the bag, i.e. facing the pivot (3) of the device, and only a multiple bag connection system (16) is attached to the upper end of the bag, and no outlet connection piece exists.

**Revendications**

1. Dispositif pour extraire des composants hétérogènes, présents dans au moins deux zones spatialement séparées, d'un liquide, en particulier de sang total stabilisé contenu dans un sachet transparent souple muni d'au moins une conduite d'évacuation (14), ce dispositif étant constitué d'une plaque avant (1) faisant partie du boîtier (11), d'une plaque de pression (2) pouvant être pivotée par rapport à la plaque avant (1), d'un dispositif d'entraînement et d'un dispositif de balayage, **caractérisé** en ce que le point de rotation (3) de la plaque de pression (2) se trouve en dessous du sachet au point d'intersection des deux surfaces de plaques, et ce à une distance supérieure à 30 % de la longueur de la plaque de pression (2), et le dispositif d'entraînement agit sur un bras de levier (12) qui est rigidement assemblé à la plaque de pression (2).

2. Dispositif selon la revendication 1, **caractérisé** en ce que le dispositif de balayage est un détecteur optique (4,10), qui actionne un mécanisme (9) pour fermer au moins un des dispositifs d'évacuation lorsqu'une couche de composant donnée atteint un niveau prédéterminé dans le sachet.

3. Dispositif selon la revendication 2, **caractérisé** en ce que le détecteur optique (4) est disposé sur la plaque avant (1) dans la région du sachet.

4. Dispositif selon la revendication 2, **caractérisé** en ce que le détecteur (10) est disposé dans la région du flexible.

5. Dispositif selon la revendication 1, **caractérisé** en ce qu'un commutateur de fin de course est en outre prévu.

6. Dispositif selon la revendication 1, **caractérisé** en ce que le dispositif d'entraînement est constitué d'un élément d'entraînement à actionnement hydraulique ou électrique.

7. Dispositif selon la revendication 6, **caractérisé** en ce que l'élément d'entraînement est disposé sur la plaque avant (1).

8. Dispositif selon l'une des revendications 1 à 7, **caractérisé** en ce qu'un capteur de pression (5) est disposé au milieu de la plaque avant (1).

9. Sachet, pourvu respectivement sur la tête et sur le fond de flexibles d'alimentation et d'évacuation et destiné à être utilisé dans un dispositif selon les revendications 1 à 8, **caractérisé** en ce qu'une conduite d'admission (13) est disposée à l'extrémité supérieure du sachet, donc loin du point de rotation (3) du dispositif, et une conduite d'évacuation (14) ainsi que le flexible de remplissage ou de prélèvement du sang (15) sont disposés sur la partie inférieure du sachet, donc près du point de rotation (3) du dispositif, et seul un système d'assemblage de sachet réutilisable (16) est fixé à l'extrémité supérieure du sachet, laquelle ne présente pas de tubulure d'échappement.

(9) , (10)

(11)

(1)

(2)

(4)

(5)

(9)

# Abb. 1

(11)

(9),(10)

(1)

(2)

(7)

(8)

(6)

(12)

(3)

# Abb. 2

(13)

(16)

(15)

(14)

# Abb. 3